# EUROPEAN PATENT SPECIFICATION

(11) **EP 4 006 529 B1**
(45) Date of publication and mention of the grant of the patent: **25.11.2026**
(21) Application number: 19939057.6
(22) Date of filing: 31.07.2019
(51) Int. Cl.: G01N 21/69, G01N 21/76

(54) **BIOMOLECULE ANALYSIS METHOD AND BIOMOLECULE ANALYSIS DEVICE**
VERFAHREN UND VORRICHTUNG ZUR ANALYSE VON BIOMOLEKÜLEN
PROCÉDÉ D'ANALYSE DE BIOMOLÉCULE ET DISPOSITIF D'ANALYSE DE BIOMOLÉCULE

(43) Date of publication of application: 01.06.2022
(73) Proprietor: Hitachi High-Tech Corporation, Minato-ku Tokyo 105-6409 (JP); F. Hoffmann-La Roche AG, 4070 Basel (CH); Roche Diagnostics GmbH, 68305 Mannheim (DE)
(72) Inventor: SAITO Toshiro, Tokyo 105-6409 (JP); IMAI Kyoko, Tokyo 105-6409 (JP); PAOLUCCI Francesco, 40126 Bologna BO (IT); MARCACCIO Massimo, 40126 Bologna BO (IT); VALENTI Giovanni, 40126 Bologna BO (IT); ZANUT Alessandra, 40126 Bologna BO (IT); WINDFUHR Michaela, 68305 Mannheim (DE); JOSEL Hans-Peter, 68305 Mannheim (DE)
(74) Representative: MERH-IP Matias Erny Reichl Hoffmann Patentanwälte PartG mbB
(86) International application number: PCT/JP2019/030018
(87) International publication number: WO 2021/019732

(56) References cited:
- WO-A2-2007/002580
- JP-A- 2008 504 528
- JP-A- 2009 139 308
- JP-A- 2009 500 020
- JP-A- 2013 542 436
- JP-A- 2016 522 417
- JP-A- 2019 512 665
- JP-A- 2019 515 251
- SENTIC MILICA ET AL: "Mapping electrogenerated chemiluminescence reactivity in space: mechanistic insight into model systems used in immunoassays", vol. 5, no. 6, 1 January 2014 (2014-01-01), United Kingdom, pages 2568 - 2572, XP093007336, ISSN: 2041-6520, Retrieved from the Internet <URL:https://pubs.rsc.org/en/content/articlepdf/2014/sc/c4sc00312h> DOI: 10.1039/C4SC00312H

## Description

### Technical Field

The present invention relates to a biomolecular analysis method and a biomolecular analyzer.

### Background Art

In recent years, a biomolecular analysis method based on an electrochemiluminescence (ECL: Electrogenerated Chemi Luminescence) method has been developed. The ECL method is a method of: generating a radical species of a reaction aid by putting a solution containing the reaction aid such as amine and a luminescent material such as a ruthenium complex in a flow cell having a working electrode, a counter electrode, and a reference electrode and applying a potential not lower than the oxidation potential of the reaction aid; forming an excited state of the luminescent material by reaction between the radical species and the luminescent material; and measuring the luminescence.

As a method related to such an ECL method for example, Patent Literature 1 discloses a technology of: forming a sandwich complex including a luminescent material modified antibody and analysis target molecules over a support comprising magnetic microparticles or the like; fixing the sandwich complex to an electrode by an external force such as a magnetic field; then generating ECL by applying a voltage; and estimating the concentration of the analysis target molecules by measuring an ECL intensity that depends on the concentration of the analysis target molecules.

Meanwhile, Nonpatent Literature 1 discloses a mechanism of generating ECL with regard to a system of using tripropylamine (TPrA) as a reaction aid and a ruthenium bipyridyl complex as a luminescent material.

Moreover, document JP 2019 515251 A describes a method for the detection of analytes in a sample by electrochemiluminescence using reagent compositions. Described are reagent compositions, reagent kits for measuring electrochemiluminescence (ECL), and electrochemiluminescence detection methods using the reagent compositions. In this document, individual branched tertiary amines, such as N,N-dipropyl-N- (sec-butyl)amine (DPIBA), are used as co-reactants for the electrochemiluminescence reaction.

WO 2007/002580 A2 describes detection devices and methods associated therewith. The detection system can be adapted to measure one or more analytes of interest possibly present in a sample through the use of binding reactions.

And JP 2009 139308 A describes, in an electrochemiluminescence method, to execute luminescence using a self-organized film by an organic metal complex represented by ferrocene.

### Citation List

### Patent Literature

Patent Literature 1: Japanese Unexamined Patent Application Publication No. Hei 4(1992)-502964

### Nonpatent Literature

Nonpatent Literature 1: Wujian Miao, Jai-Pil Choi, and Allen J. Bard, "The Tris(2,2'-bipyridine)ruthenium(II), (Ru(bpy)3^2+)/Tri-n-propylamine (TPrA) System Revisited - A New Route Involving TPrA·+ Cation Radicals", Journal of American Chemical Society 124(48), (2002), pp 14478-14485

### Summary of Invention

### Technical Problem

A biomolecular analysis method based on an ECL method is researched actively in order to be used for the quantification of blood markers for the early diagnosis of cancer or heart disease, and is being applied to clinical diagnosis partially. In such a field, further enhanced detection sensitivity is required.

The present invention has been established in view of the above situation and an object of the present invention is to provide a biomolecular analysis method and a biomolecular analyzer which are capable of enhancing detection sensitivity by increasing the luminous efficiency **of ECL.**

### Solution to Problem

There are provided biomolecular analysis methods and biomolecular analyzers having the features of the respective independent claims. The dependent claims relate to preferred embodiments.

### Advantageous Effects of Invention

Detection sensitivity can be enhanced by increasing the luminous efficiency of **ECL.**

### Brief Description of Drawings

Fig. 1 is a view extracting and schematically showing a substantial part of a biomolecular analyzer according to First Embodiment.
**Fig.** 2 is a view explaining the basic principle of electrochemiluminescence.
**Fig.** 3 is a graph showing a relationship between a diameter of magnetic microparticles and a number of ruthenium complex molecules per unit surface area.
Fig. 4 is a view showing an example of observing luminescence intensity of ECL..
Fig. 5 is a graph showing a relationship between a diameter of magnetic particles and a luminescence intensity of ECL.
Fig. 6 is a view showing a measurement system used for investigating a relationship between a distance between a microelectrode and a surface to which a luminescence unit is fixed and an ECL intensity.
Fig. 7 is a graph showing a relationship between a distance between a luminescence unit fixed substrate and an electrode and an ECL intensity.
Fig. 8 is a graph showing a relationship between the radicals travelling time and an ECL intensity.
Fig. 9 is a view explaining a reaction process of electrochemiluminescence according to First Embodiment.
Fig. 10 is a graph showing an ECL intensity enhancement when the quantity of a second amine compound added to a first amine compound is changed.

### Description of Embodiments

Embodiments according to the present invention are explained hereunder in reference to drawings.

### <First Embodiment>

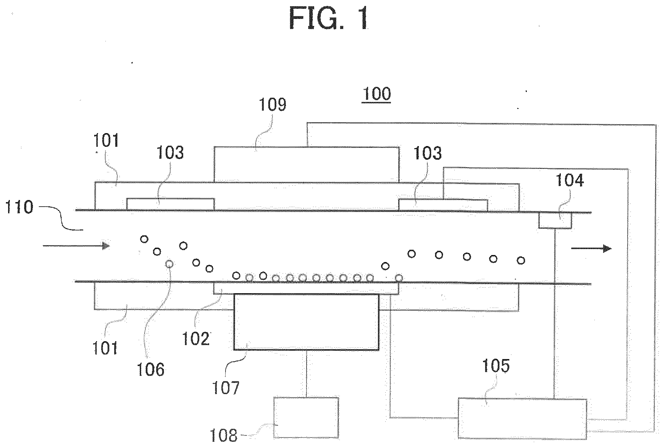

First Embodiment, useful for understanding the invention, is explained hereunder in detail in reference to Figs. 1 to 9.

Fig. 1 is a view extracting and schematically showing a substantial part of a biomolecular analyzer according to the present embodiment.

In Fig. 1, a biomolecular analyzer 100 has: a flow cell 101; a working electrode 102, a counter electrode 103, and a reference electrode 104 which are arranged in the interior of the flow cell 101; and a controller 105 carrying out analysis processing of making a solution (described later in detail) containing a test object, magnetic beads 106 (a magnetic support) over the surfaces of which a complex including an antibody that is labeled with a luminescent agent and recognizes the test object is formed, and a reaction aid to assist reaction of the luminescent agent is made to flow into a flow channel 110 of the flow cell 101, capturing the magnetic beads 106 over the working electrode 102 by a magnetic field generated by a magnet 107 (magnetic force generator) that is shifted to the vicinities of the flow cell 101 and the working electrode 102 by a magnet position controller 108, making the luminescent agent to illuminate by applying a voltage at the working electrode 102 by the controller 105, and measuring an amount of luminescence of the luminescent agent by an electrochemiluminescence detector 109. The controller 105 is a device that controls the overall behavior of the biomolecular analyzer 100, carries out the control of the position of the magnet 107 by the magnet position controller 108, controls the voltages applied to respective electrodes acting as a potentiostat (voltage controller), controls the analysis processing of a detection result of the electrochemiluminescence detector 109, and others.

The present embodiment analyzes biomolecules in a specimen by carrying out capturing and labeling by using immune recognition, and explains particularly basing on the use of magnetic microparticles as a support for a sandwich complex formed by the immune recognition. By using magnetic microparticles as a support like the present embodiment, it is possible to: magnetically separate a reactant and an unreacted material; and carry out high-speed and high-sensitivity analysis.

On this occasion, a sandwich complex comprising a capture antibody that links the magnetic microparticles with the target analytical biomolecules, the biomolecules, a detection antibody that linked to the label material that link the biomolecules. The resulting sandwich complex is fixed to the magnetic microparticles. The magnetic microparticles are captured on an electrode by an external magnetic force or the like. As a result, a label material such as a ruthenium bipyridyl complex is not directly oxidized or reduced by the electrode. A so-called reaction aid that is oxidized or reduced by the electrode and reflects the influence to the label material therefore is required. In the present embodiment, an active species of a label material is formed by using tripropylamine (TPrA) as the reaction aid and making a radical species of the reaction aid formed over the electrode surface react with the label material and electrochemiluminescence (ECL: Electrogenerated Chemi Luminescence) is generated during deactivation processing.

Firstly, the basic principle of electrochemiluminescence (ECL: Electrogenerated Chemi Luminescence) generation in the present embodiment is explained.

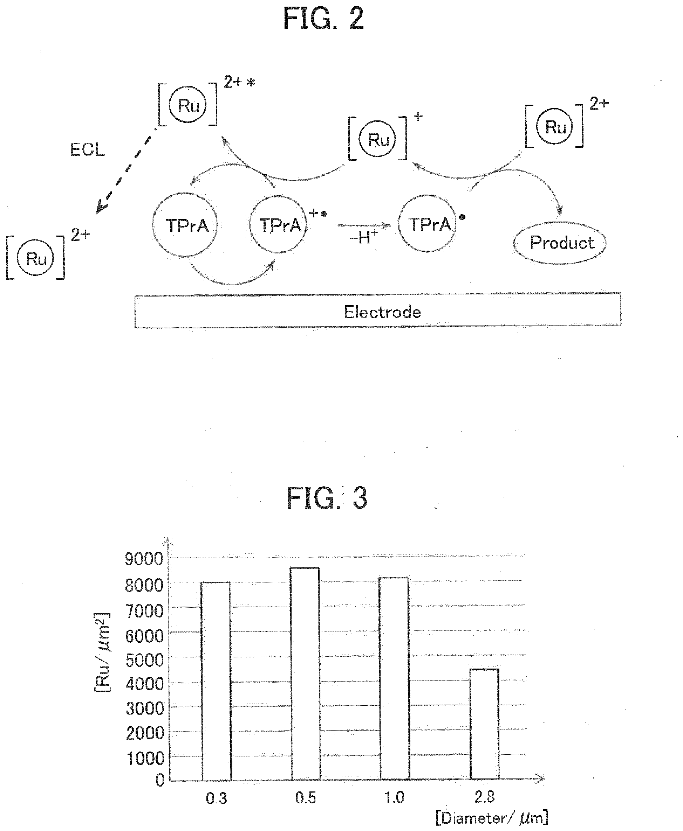

Fig. 2 is a view explaining the basic principle of electrochemiluminescence (ECL) generation.

Fig. 2 shows a system of using tripropylamine (TPrA) as a reaction aid and a ruthenium bipyridyl complex (Ru) as a luminescent material.

In Fig. 2, firstly TPrA is oxidized over an electrode and a TPrA cation radical is generated. Successively, a TPrA radical (neutral) is generated by proton abstraction reaction of the TPrA cation radical. A ruthenium bipyridyl complex (2+) and the TPrA radical (neutral) react and a ruthenium bipyridyl complex (1+) is generated. Further, an excited state of the ruthenium bipyridyl complex (2+) is formed by the reaction of the TPrA cation radical and the ruthenium bipyridyl complex (1+) and ECL is generated when the ruthenium bipyridyl complex (2+) illuminates.

It is thought that: the luminous efficiency of ECL is related to the probability of radical species reaching a ruthenium complex over magnetic microparticles within the lifetime of the radical species in a reaction aid; and the luminous efficiency can be enhanced by increasing the reaching probability. In studying a method of increasing a luminous probability therefore, firstly the following verification is carried out in order to obtain a relationship between a diameter of magnetic microparticles and a luminescence intensity of ECL.

Fig. 3 is a graph showing a relationship between a diameter of magnetic microparticles and a number of ruthenium complex molecules per unit surface area.

In obtaining the relationship of Fig. 3, firstly magnetic microparticles, the surfaces of. which are coated with streptavidin, 2.8 µm in diameter (Dynabeads M-270 Streptavidin made by Thermo Fisher Scientific), 1 µm in diameter (Dynabeads MyOne Streptavidin T1 made by Thermo Fisher Scientific), 0.5 µm in diameter (Bio-MasterBeads Streptavidin 500nm made by ADEMTECH), and 0.3 µm in diameter (Bio-Adembeads Streptavidin 300nm made by ADEMTECH) are prepared. Further, a biotinylated antibody labeled with a ruthenium bipyridyl complex is manufactured by modifying an antibody (ab403 made by Abcam) with biotin of Biotin Labeling Kit-SH made by DOJINDO LABORATORIES and then making the antibody react with ruthenium bipyridyl complex labeled molecules (bis(2,2'-bipyridine)-4'-methyl-4-carboxybipyridine-ruthenium N-succinimidyl ester-bis (hexafluorophosphate)) made by Sigma-Aldrich). By making the biotinylated antibody labeled with the ruthenium bipyridyl complex react with the magnetic microparticles coated with streptavidin, magnetic microparticles of respective sizes to which the ruthenium bipyridyl complex is fixed are obtained.

A quantity of a ruthenium complex over magnetic microparticles is determined with an inductively coupled plasma mass spectrometer. A specimen is prepared by adding a nitric acid/hydrochloric acid mixed solution to a predetermined quantity of a magnetic microparticle dispersing liquid and dissolving magnetic microparticles with an acid digestion system at 80°C. An internal standard method is adopted as measurement method using the mass spectrometer and Y (20 ng/ml) is used as an internal standard element. As a concentration of the magnetic microparticles, a value stated in a data sheet provided by each maker is adopted. From those values, the relationship shown in Fig. 3 is obtained.

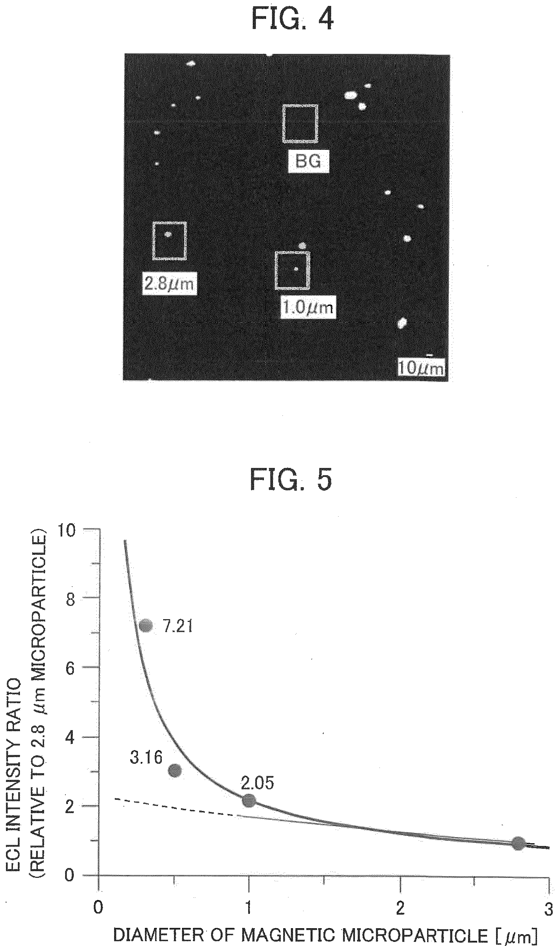

Fig. 4 is a view showing an example of observing luminescence intensity of ECL. Further, Fig. 5 is a graph showing a relationship between a diameter of magnetic particles and a luminescence intensity of ECL.

In the measurement of a luminescence intensity of ECL, the luminescence intensity of every single magnetic microparticle is measured for the reasons that: if magnetic microparticles are observed as a group, the magnetic microparticles overlap with each other and are fixed to a substrate and that affects a luminescence intensity; a luminescence intensity depends largely on the number of magnetic microparticles over a substrate and the number is hardly grasped precisely and corrected.

Specifically, a flow cell in which a working electrode, a counter electrode, and a reference electrode (Ag/AgCl) are arranged at a distance of 0.5 mm between electrodes is prepared and filled with a solution containing magnetic microparticles of respective sizes (diameters) to which a ruthenium bipyridyl complex is fixed. In order to measure luminescence emitted from magnetic microparticles of different sizes under the same conditions, the flow cell is filled with a pair of magnetic microparticles 2.8 µm and 0.3 µm in diameter, a pair of magnetic microparticles 2.8 µm and 0.5 µm in diameter, and a pair of magnetic microparticles 2.8 µm and 1 µm in diameter respectively and differences in luminescence intensity between two sizes in the respective pairs are measured. In the measurement, the flow cell is placed under a microscope, luminescence is collected with an objective lens (×100), and then a luminescent image is obtained with an EM-CCD (Electro Multiplying Charge Coupled Device, 9100-13, made by Hamamatsu Photonics K.K.). An image of observing magnetic microparticles 2.8 µm and 1 µm in diameter is shown in Fig. 4 as an example. Image processing software ImageJ is used for numerical processing, an integrated value of intensities per pixel in a certain area surrounding a bright spot is obtained, and a signal value is calculated by subtracting the region where the bright spot does not exist as a background (BG).

Further, a rate of a signal value to a number of ruthenium complex molecules is calculated and further a ratio of the rate to the rate of the magnetic microparticle 2.8 µm in diameter is obtained. As shown in Fig. 5, the ECL efficiency increases significantly as the diameter of a magnetic microparticle reduces and the ECL efficiency is about twice in the case of 1.0 µm and about seven times in the case of 0.3 µm in diameter in comparison with the case of 2.8 µm in diameter. It has therefore been found that ECL can be increased and luminous efficiency can be enhanced by reducing the diameter of a magnetic microparticle used as a support. In particular, a higher luminous efficiency can be expected by using a magnetic microparticle 1 µm or lower in diameter.

In Fig. 5, as an example comparable to the verification result according to the present embodiment, the relationship between a microelectrode-substrate (to which a ruthenium complex is fixed) distance and an ECL intensity in prior art (Nonpatent Literature 1) is represented by the solid line and the relationship estimated in the range of the distance of 1 µm or lower is represented by the extrapolation line (dotted line).

As it is obvious from Fig. 5, in the present embodiment, significantly unlike the results estimated from prior art, ECL efficiency improves largely as a distance between a ruthenium complex and an electrode reduces to 1 µm or lower. It shows, in other words, that ECL efficiency can improve far better by using a magnetic microparticle of 1 µm or lower in diameter.

Further, in order to verify the effect of downsizing a magnetic microparticle here, the relationship between a distance between a microelectrode and a substrate to which a ruthenium complex is fixed and an ECL intensity is investigated by using a micromanipulator positioned in the proximity of an emitting surface.

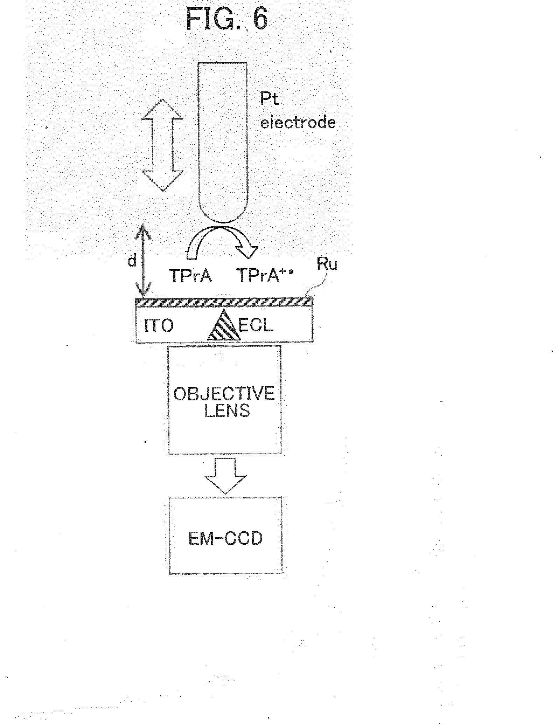

Fig. 6 is a view showing a measurement system used for investigating a relationship between a distance between a microelectrode and a substrate to which a ruthenium complex is fixed and an ECL intensity.

As shown in Fig. 6, a micromanipulator(Scanning electrochemical microscopy SECM:.CHI910B, made by CH Instruments, Inc.) is used as the measurement system, a platinum wire (Pt electrode) of 1.3 mm in diameter is used as the working electrode, a platinum wire is used as the counter electrode, and Ag/AgCl (3M) is used as the reference electrode. Further, an ITO (tin oxide-doped indium oxide) glass substrate to which a ruthenium bipyridyl complex is fixed is used as the substrate. The ruthenium bipyridyl complex is fixed by coating the surface of the ITO glass substrate with aminosilane beforehand and making ruthenium bipyridyl complex labeled molecules (bis(2,2'-bipyridine)-4'-methyl-4-carboxybipyridine-ruthenium N-succinimidyl ester-bis(hexafluorophosphate)) made by Sigma-Aldrich) react over the surface.

Further, a phosphoric acid buffer (pH 6.8) in which TPrA of 180 mM is dissolved as a reaction aid is put into a very small container; the counter electrode, the reference electrode, and the ITO glass substrate are immersed; further the platinum microelectrode is inserted into the solution; and the insertion position is controlled with the SECM. A through hole of about 3 mm in diameter is formed at the bottom of the very small container, the solution is prevented from leaking with an O-ring, and the very small container is placed over a specimen table of an inverted microscope. ECL passes through the hole and is collected with an objective lens (×4) and then a luminescent image is obtained with the EM-CCD (9100-13 made by Hamamatsu Photonics K.K.). When the platinum microelectrode is brought close to the ITO substrate while electric current is monitored and the electrode touches the ITO substrate, the area of the electrode increases extremely and hence spiky increase of electric current is observed. The electrode position is defined as a standard (distance 0 (zero)). The electrode position is changed so that the distance from the substrate may be 0.1 µm, 0.2 µm, 0.3 µm, 0.5 µm, 0.7 µm, 1.0 µm, 1.5 µm, 2.0 µm, and 2.8 µm, further the voltage is swept from 0 V to 1.4 V, and ECL images on those occasions are obtained.

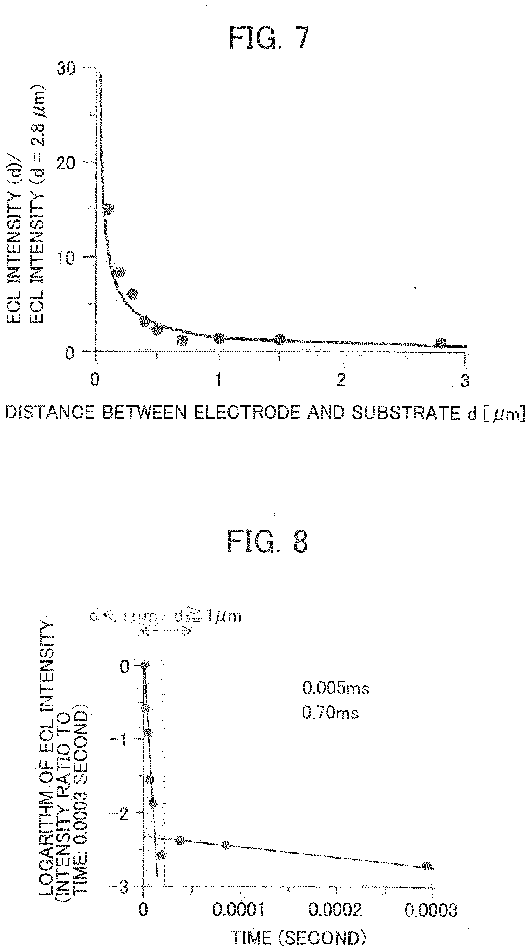

Fig. 7 is a graph showing a relationship between a distance between a ruthenium complex fixed substrate and an electrode and an ECL intensity.

In Fig. 7, an ECL intensity is shown by being standardized with the ECL intensity at a distance of 2.8 µm. In this verification, it is verified that, as the position of an electrode comes close to an ITO substrate, an ECL image shines brightly in response to the voltage sweep. In Fig. 7, it is obvious that the ECL intensity increases rapidly when the distance between the substrate and the electrode is 1 µm or less similarly to the relationship between the diameter of the magnetic moicroparticle and the ECL intensity shown in Fig. 6. Further, it is obvious that the rate of the increase matches very well with the case shown in Fig. 6.

As stated above, the relationship between a diameter of a magnetic microparticle and an ECL intensity and the relationship between a distance between a ruthenium complex and an electrode and an ECL intensity are correlated very well. Further, it is found that the relationship is entirely different from a result estimated through prior art. Thus, these results show that ECL occurs under a different mechanism when a ruthenium complex is located at a position close (< 1 µm) to or far (> 1 µm) from an electrode. Moreover, it is considered that a factor of improving an ECL intensity when a ruthenium complex is located at a position close (< 1 µm) to an electrode is that the quantity of a radical species existing in the vicinity of the electrode is large. Meanwhile, the lifetime of a radical species is considered to be very short because an ECL intensity lowers (in other words, the effect of a radical species disappears) at a position far from an electrode.

Given that radical species generated over an electrode are dispersed by diffusion, a distance d to reach at a diffusion coefficient D and a time t is given by the following Expression 1. $t = d 2 / \left(36D\right)$ Expression 1

Fig. 8 is a graph showing a relationship between a time elapsed since the generation of a radical species and an ECL intensity.

In Fig. 8, the diffusion coefficient D is set at 5×10^-6 (cm²/s), the distance d is converted to an arrival time, the ECL intensity is standardized by a maximum value, and then logarithm is taken. In Fig. 8, it is obvious that there are two components, a long-lived component and a short-lived component. The half-life time of the long-lived component (region where an arrival distance is 1 µm or more) is 0.3 to 0.7 ms. The half-life time is a value very close to the lifetime (0.2 ms) of a cation radical of TPrA determined by electron spin resonance in prior art. In contrast, the lifetime of the short-lived component is about 5 µs and it is obvious that a radical species of a short lifetime is involved.

Further, if a potential of generating a maximum ECL intensity is examined, the potential is about 1.4 V (relative to Ag/AgCl) and the radical species generated at the potential is considered to be generated by the oxidation reaction of TPrA. The elementary process of reaction of a cation radical species in an aliphatic amine has long been known since old times, for example breakage of a bond between a carbon atom and a nitrogen atom occurs from a cation radical generated by oxidation reaction over an electrode, and the generated neutral radical (unpaired electrons are localized to the nitrogen atom) has a very short lifetime (refer to Journal of American Chemical Society 34, (1969), pp 1821-1826 and the like, for example). Moreover, the neutral radical abstracts a proton from another aliphatic amine molecule and a neutral radical (unpaired electrons are localized to the α carbon atom) of the other aliphatic amine molecule is generated. Resultantly, the neutral radical (unpaired electrons are localized to the α carbon atom) of TPrA is generated and hence a radical species indispensable for producing an exited state (refer to Fig. 2) of a ruthenium complex is generated more in a new reaction process (hereunder referred to as second reaction process) different from a reaction process (hereunder referred to as first reaction process) in the basic principle. Since the neutral radical (unpaired electrons are localized to the nitrogen atom) of anintermediate product has a very short lifetime, the new reaction process (second reaction process) is considered to work effectively only in the vicinity of an electrode.

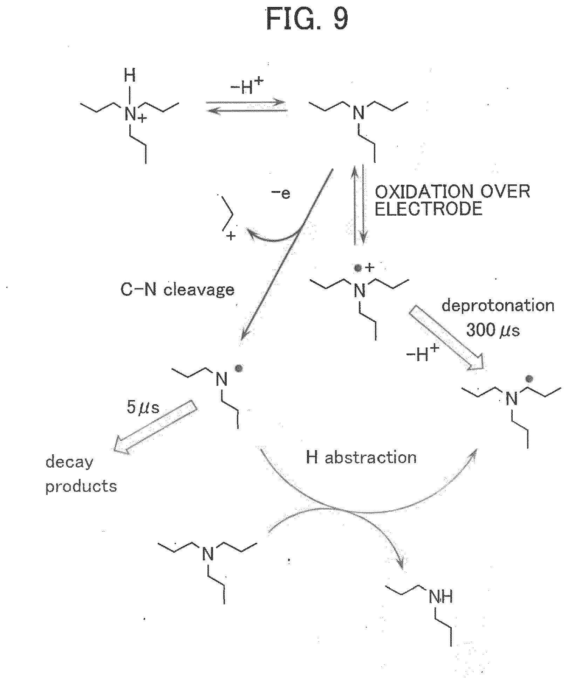

Fig. 9 is a view explaining a reaction process of electrochemiluminescence according to the present embodiment.

In a region near an electrode, a neutral radical is produced not only through a first reaction process of abstracting a proton from a cation radical of TPrA and producing a neutral radical (unpaired electrons are localized to an α carbon atom) but also through a second reaction process of cutting a C-N bond along with oxidation over the electrode, producing a neutral radical (unpaired electrons are localized to a nitrogen atom), and producing a neutral radical (unpaired electrons are localized to an α carbon atom) by abstracting hydrogen from another aliphatic amine molecule and hence an exited state of a ruthenium complex is formed at a higher efficiency and an ECL intensity increases.

The ECL mechanism in the second reaction process works effectively and an ECL intensity increases significantly as long as the diameter of a magnetic microparticle is 1 µm or less (more desirably 0.5 µm or less in diameter). In other words, by using a magnetic microparticle 1 µm or less in diameter (more desirably 0.5 µm or less in diameter), ECL is generated through the new reaction process and hence high sensitivity can be achieved. Further, detection sensitivity can be adjusted by adjusting the particle size of a used magnetic microparticle. It is therefore possible to make analysis with an optimum sensitivity by using magnetic particles having at least two kinds of particle sizes as a support and selecting a magnetic particle used in accordance with the kind of a test object.

### <Second Embodiment>

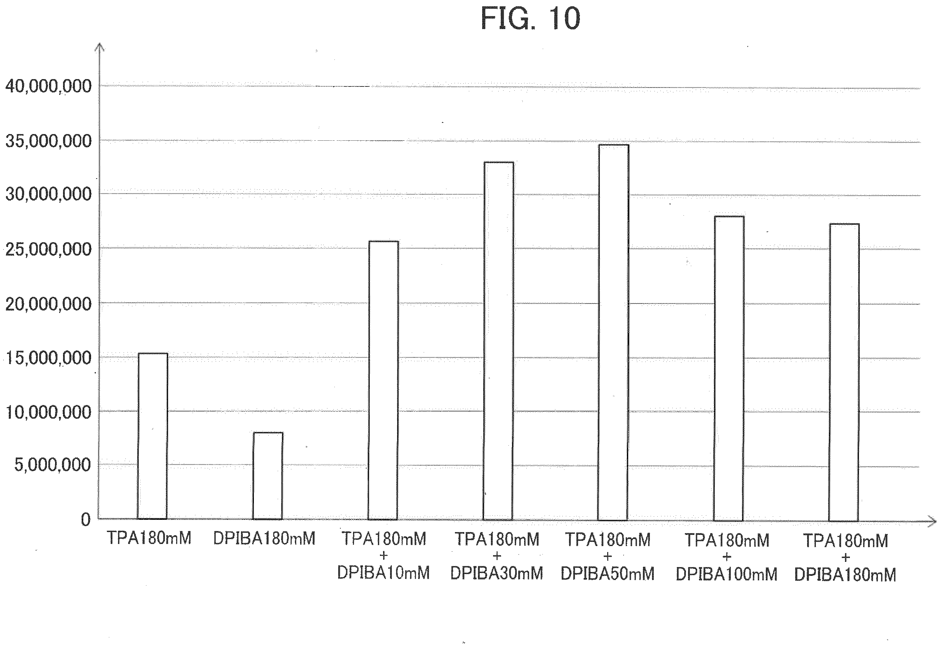

Second Embodiment, according to the present invention, is explained hereunder in detail in reference to Fig. 10.

The present embodiment is, in view of the concern that it takes time to collect magnetism and that a throughput lowers when a magnetic microparticle with small diameter is used as shown in First Embodiment, a method of increasing detection sensitivity by way of a new reaction process (hereunder referred to as third reaction process) conforming to the second reaction process by adding a specific amine compound (hereunder referred to as second reaction aid or second amine compound) that is different from the reaction aid (hereunder referred to as first reaction aid or first aminecompound) in the first reaction process even when a magnetic microparticle of 1 µm or more (more desirably about 2.8 µm) in diameter that is easily handled is used as a magnetic microparticle.

In the present embodiment according to the invention, specifically, an amine compound (aliphatic amine) where C-N bond cleavage is induced by electrochemical oxidation is added as a second amine compound in a solution. By adding the second amine compound, a secondary amine radical that has lost an alkyl group is formed, the secondary amine radical abstracts hydrogen from an amine of a first reaction aid, and a neutral radical of the first reaction aid is produced (hereunder referred to as third reaction process). In other words, a neutral radical is formed through the third reaction process in addition to the first reaction process of forming the neutral radical by way of the cation radical of the amine of the first reaction aid oxidized over an electrode.

In this way, the present invention, by being configured so as to go through a second reaction process in addition to a first reaction process, can obtain the effects of (1). increasing the quantity of a produced neutral radical species, (2) increasing the generation rate of a neutral radical more in the second reaction process than in the first reaction process, and (3) being able to form a neutral radical species of a first reaction aid while the quantity of a cation radical is maintained because it does not go through the cation radical of the first reaction aid that is indispensable for ECL. In other words, by adding a second amine compound, the quantity of ECL can be increased.remarkably.

Here, conditions necessary for a second amine compound are that a C-N bond cleavage is induced by the electrochemical oxidation and the stability of a carbocation formed after the C-N bond cleavage is ensured. To that end, the second amine compound preferably has a structure suitable for delocalization of a charge and has an unsaturated bond. In other words, as a second amine compound (second reaction aid), 1-allylpiperidine or 1-allyl pyrrolidine is named for example.

Further, as a structure advantageous to the stability of a carbocation, a structure of containing a hydroxyl group or a halogen group in a dissociating carbon chain is desirable.

Furthermore, conditions necessary for a second amine compound are that the second amine compound is likely to oxidize over an electrode and a low energy highest occupied molecular orbital (HOMO) is advantageous. To that end, an amine having a branch structure is desirable. That is, as a second amine compound (secondreaction aid), named for example is diisobutyl-isobutylamine, diisobutyl-propylamine, N,N-dipropyl-N-(secobutyl)amine, or N,N-di(secobutyl)-N-propylamine.

Here, as a first amine compound (first reaction aid), TPrA showing a high luminescence intensity is preferably used and, as an aliphatic amine, an aliphatic amine such as a first, second, or third alkylamine in which each alkyl group has a carbon atom number of 1 to 4 or a substituted aliphatic amine may be used.. Specifically, as a first amine compound, used for example may be triethanolamine, triethylamine, 1,4-diazabicyclo(2,2,2)-octane, 1-piperidine ethanol, 1,4-piperazine-bis-(ethane-sulfonic acid), or triisopropylamine.

Fig. 10 is a graph showing an ECL intensity changing when the quantity of a second amine compound added to a first amine compound is changed. In Fig. 10, TPrA is used as the first amine compound, dipropyl-isobutylamine (DPIBA) is used as the second amine compound, and an ECL intensity is measured while the mixing ratio is changed.

As shown in Fig. 10, in comparison with the case of TPrA only (180 mM), the ECL intensity increases as DPIBA is added more and shows the highest value (maximum value) when the quantity of the added DPIBA is 50 mM. Then, as the quantity of the added DPIBA is further increased, the ECL intensity turns to reduce.

Further, in the case of DPIBA only (180 mM), the ECL intensity is extremely weak and hence the function as a reaction aid is estimated to be very low when only DPIBA is used.

Meanwhile, the ECL intensity increases dramatically by adding an amine compound (DPIBA 10 mM) that has a low reaction support function alone to TPrA (180 mM). It is therefore estimated that, by adding DPIBA to TPrA, the ECL intensity increases because the TPrA neutral radical formation caused during a third reaction process conforming to a second reaction process contributes largely in addition to the reaction of TPrA alone.

Further, it is estimated that, if DPIBA of 50 mM or more is added, the oxidation reaction of TPrA and the oxidation reaction of DPIBA occur competitively, resultantly the influence of DPIBA that works poorly as a reaction aid appears undesirably during the reaction process, and the ECL intensity finally reduces.

As stated above, by using at least two kinds of aliphatic amines, that is, an aliphatic amine (first reaction aid) that has only a linear alkyl chain and is represented by TPrA and an aliphatic amine (second reaction aid) that causes a nitrogen-carbon bond to dissociate in succession to oxidation reaction over an electrode and is represented by DPIBA, as reaction aids, it is possible to increase the luminescence intensity of ECL remarkably and achieve a higher sensitivity.

Further, detection sensitivity can be adjusted by adjusting the content ratio of at least two kinds of aliphatic amines, that is, an aliphatic amine (first reaction aid) that has only a linear alkyl chain and an aliphatic amine (second reaction aid) that causes a nitrogen-carbon bond to dissociate in succession to oxidation reaction over an electrode, in accordance with the kind of a test object and hence analysis can be performed with a sensitivity optimum to detection.

### <Additional Remark>

Here, the present invention is not limited to the aforementioned embodiments and includes various modified examples and combinations within the scope of the appended claims.

### List of Reference Signs

101...Flow cell
102...Working electrode
103... Counter electrode
104...Reference electrode
105... Controller
106...Magnetic beads (Magnetic support)
107...Magnet (Magnetic force generator)
108...Magnet position controller
109...Electrochemiluminescence detector
110...Flow channel

## Claims

1. A biomolecular analysis method comprising:
an inflow process of causing a solution to flow in a flow cell (101), the solution containing a test object, a magnetic support (106) on the surface of which a complex including an antibody that is labeled with a luminescent agent and recognizes the test object is formed, and a first aliphatic amine that has only a linear alkyl chain and a second aliphatic amine as first and second reaction aids to assist reaction of the luminescent agent,
wherein the second aliphatic amine has at least an alkyl chain having a branch structure, or the second aliphatic amine has an unsaturated bond in an alkyl chain, or the second aliphatic amine has a hydroxyl group or a halogen atom in an alkyl chain;
a capture process of capturing the magnetic support (106) over a working electrode (102) by a magnetic field generated by a magnetic force generator (107);
a luminescent process of making the luminescent agent illuminate by applying a voltage to the working electrode (102); and
a measurement process of measuring an amount of luminescence of the luminescent agent,
wherein the luminescent process includes:
luminescence caused by action of a first neutral radical and a second neutral radical on the luminescent agent,
wherein the first neutral radical is produced from the first aliphatic amine which is the reaction aid through a cation radical;
wherein the second neutral radical is produced by the dissociation of a nitrogen-carbon bond in succession to oxidation reaction over an electrode of the second aliphatic amine and not through a cation radical, and
wherein the second neutral radical further interacts with the first aliphatic amine to produce additional first neutral radicals.

2. A biomolecular analysis method according to Claim 1, wherein the first aliphatic amine is tripropylamine.

3. A biomolecular analysis method according to Claim 1, wherein the second aliphatic amine is dipropyl isobutylamine.

4. A biomolecular analysis method according to Claim 1, wherein the second aliphatic amine is diisopropyl ethylamine.

5. A biomolecular analysis method according to Claim 1, wherein the second aliphatic amine is 1-allylpiperidine.

6. A biomolecular analysis method according to Claim 1, wherein the second aliphatic amine is 1-allyl pyrrolidine.

7. A biomolecular analysis method according to Claim 1, wherein a content ratio of the first aliphatic amine and the second aliphatic amine is adjusted in accordance with a kind of the test object.

8. A biomolecular analyzer comprising:
a flow cell (101);
a working electrode (102), a counter electrode (103), and a reference electrode (104) which are arranged in the interior of the flow cell (101); and
a controller (105) of carrying out analysis processing of: causing a solution to flow in the flow cell (101), the solution containing a test object, a magnetic support (106) over the surface of which a complex including an antibody that is labeled with a luminescent agent and recognizes the test object is formed, and a first aliphatic amine that has only a linear alkyl chain and a second aliphatic amine as first and second reaction aids to assist reaction of the luminescent agent,
wherein the second aliphatic amine has at least an alkyl chain having a branch structure, or the second aliphatic amine has an unsaturated bond in an alkyl chain, or the second aliphatic amine has a hydroxyl group or a halogen atom in an alkyl chain;
capturing the magnetic support (106) over the working electrode (102) by a magnetic field generated by a magnetic force generator (107); making the luminescent agent illuminate by applying a voltage to the working electrode (102); and measuring an amount of luminescence of the luminescent agent with a measuring device,
wherein the solution contains a first neutral radical and a second neutral radical and acts on luminescence of the luminescent agent,
wherein the first neutral radical is produced from the first aliphatic amine which is the reaction aid through a cation radical and acts on luminescence of the luminescent agent,
wherein the second neutral radical is produced by the dissociation of a nitrogen-carbon bond in succession to oxidation reaction over an electrode of the second aliphatic amine and not through a cation radical,
wherein the second neutral radical further interacts with the first aliphatic amine to produce additional first neutral radicals.

## Patentansprüche

1. Biomolekül-Analyseverfahren, umfassend:
einen Einströmvorgang, bei dem eine Lösung in eine Durchflusszelle (101) eingeleitet wird, wobei die Lösung ein Testobjekt, einen magnetischen Träger (106), auf dessen Oberfläche ein Komplex gebildet ist, der einen Antikörper umfasst, der mit einem Lumineszenzmittel markiert ist und das Testobjekt erkennt, sowie ein erstes aliphatisches Amin, das nur eine lineare Alkylkette aufweist, und ein zweites aliphatisches Amin als erste und zweite Reaktionshilfe zum Unterstützen einer Reaktion des Lumineszenzmittels enthält,
wobei das zweite aliphatische Amin zumindest eine Alkylkette mit einer verzweigten Struktur aufweist oder das zweite aliphatische Amin eine ungesättigte Bindung in einer Alkylkette aufweist oder das zweite aliphatische Amin eine Hydroxylgruppe oder ein Halogenatom in einer Alkylkette aufweist;
einen Einfangvorgang, bei dem der magnetische Träger (106) über einer Arbeitselektrode (102) durch ein von einem Magnetkrafterzeuger (107) erzeugtes Magnetfeld eingefangen wird;
einen Lumineszenzvorgang, bei dem das Lumineszenzmittel durch Anlegen einer Spannung an die Arbeitselektrode (102) zur Lumineszenz angeregt wird; und
einen Messvorgang, bei dem eine Lumineszenzmenge des Lumineszenzmittels gemessen wird,
wobei der Lumineszenzvorgang Folgendes umfasst:
eine Lumineszenz, die durch Einwirkung eines ersten neutralen Radikals und eines zweiten neutralen Radikals auf das Lumineszenzmittel hervorgerufen wird,
wobei das erste neutrale Radikal aus dem ersten aliphatischen Amin, das die Reaktionshilfe ist, über ein Radikalkation erzeugt wird;
wobei das zweite neutrale Radikal durch die Dissoziation einer Stickstoff-Kohlenstoff-Bindung nach einer Oxidationsreaktion des zweiten aliphatischen Amins über einer Elektrode und nicht über ein Radikalkation erzeugt wird, und
wobei das zweite neutrale Radikal ferner mit dem ersten aliphatischen Amin wechselwirkt, um zusätzliche erste neutrale Radikale zu erzeugen.

2. Biomolekül-Analyseverfahren nach Anspruch 1, wobei das erste aliphatische Amin Tripropylamin ist.

3. Biomolekül-Analyseverfahren nach Anspruch 1, wobei das zweite aliphatische Amin Dipropylisobutylamin ist.

4. Biomolekül-Analyseverfahren nach Anspruch 1, wobei das zweite aliphatische Amin Diisopropylethylamin ist.

5. Biomolekül-Analyseverfahren nach Anspruch 1, wobei das zweite aliphatische Amin 1-Allylpiperidin ist.

6. Biomolekül-Analyseverfahren nach Anspruch 1, wobei das zweite aliphatische Amin 1-Allylpyrrolidin ist.

7. Biomolekül-Analyseverfahren nach Anspruch 1, wobei ein Mengenverhältnis des ersten aliphatischen Amins und des zweiten aliphatischen Amins in Abhängigkeit von einer Art des Testobjekts eingestellt wird.

8. Biomolekül-Analysevorrichtung, umfassend:
eine Durchflusszelle (101);
eine Arbeitselektrode (102), eine Gegenelektrode (103) und eine Referenzelektrode (104), die im Inneren der Durchflusszelle (101) angeordnet sind; und
eine Steuerung (105) zum Ausführen einer Analyseverarbeitung, umfassend: Einleiten einer Lösung in die Durchflusszelle (101), wobei die Lösung ein Testobjekt, einen magnetischen Träger (106), über dessen Oberfläche ein Komplex gebildet ist, der einen Antikörper umfasst, der mit einem Lumineszenzmittel markiert ist und das Testobjekt erkennt, sowie ein erstes aliphatisches Amin, das nur eine lineare Alkylkette aufweist, und ein zweites aliphatisches Amin als erste und zweite Reaktionshilfe zum Unterstützen einer Reaktion des Lumineszenzmittels enthält,
wobei das zweite aliphatische Amin zumindest eine Alkylkette mit einer verzweigten Struktur aufweist oder das zweite aliphatische Amin eine ungesättigte Bindung in einer Alkylkette aufweist oder das zweite aliphatische Amin eine Hydroxylgruppe oder ein Halogenatom in einer Alkylkette aufweist;
Einfangen des magnetischen Trägers (106) über der Arbeitselektrode (102) durch ein von einem Magnetkrafterzeuger (107) erzeugtes Magnetfeld; Anregen des Lumineszenzmittels zur Lumineszenz durch Anlegen einer Spannung an die Arbeitselektrode (102); und Messen einer Lumineszenzmenge des Lumineszenzmittels mit einer Messvorrichtung,
wobei die Lösung ein erstes neutrales Radikal und ein zweites neutrales Radikal enthält und auf die Lumineszenz des Lumineszenzmittels einwirkt,
wobei das erste neutrale Radikal aus dem ersten aliphatischen Amin, das die Reaktionshilfe ist, über ein Radikalkation erzeugt wird und auf die Lumineszenz des Lumineszenzmittels einwirkt,
wobei das zweite neutrale Radikal durch die Dissoziation einer Stickstoff-Kohlenstoff-Bindung nach einer Oxidationsreaktion des zweiten aliphatischen Amins über einer Elektrode und nicht über ein Radikalkation erzeugt wird,
wobei das zweite neutrale Radikal ferner mit dem ersten aliphatischen Amin wechselwirkt, um zusätzliche erste neutrale Radikale zu erzeugen.

## Revendications

1. Procédé d'analyse biomoléculaire, comprenant :
un processus d'introduction consistant à faire circuler une solution dans une cellule à circulation (101), la solution contenant un objet de test, un support magnétique (106) à la surface duquel est formé un complexe comprenant un anticorps qui est marqué par un agent luminescent et qui reconnaît l'objet de test, ainsi qu'une première amine aliphatique qui ne comporte qu'une chaîne alkyle linéaire et une seconde amine aliphatique en tant que premier et second auxiliaires de réaction destinés à favoriser une réaction de l'agent luminescent,
dans lequel la seconde amine aliphatique comporte au moins une chaîne alkyle ayant une structure ramifiée, ou la seconde amine aliphatique comporte une liaison insaturée dans une chaîne alkyle, ou la seconde amine aliphatique comporte un groupe hydroxyle ou un atome d'halogène dans une chaîne alkyle ;
un processus de capture consistant à capturer le support magnétique (106) au-dessus d'une électrode de travail (102) au moyen d'un champ magnétique généré par un générateur de force magnétique (107) ;
un processus de luminescence consistant à exciter la luminescence de l'agent luminescent en appliquant une tension à l'électrode de travail (102) ; et
un processus de mesure consistant à mesurer une quantité de luminescence de l'agent luminescent,
dans lequel le processus de luminescence comprend :
une luminescence provoquée par l'action d'un premier radical neutre et d'un second radical neutre sur l'agent luminescent,
dans lequel le premier radical neutre est produit à partir de la première amine aliphatique, qui est l'auxiliaire de réaction, par l'intermédiaire d'un cation radical ;
dans lequel le second radical neutre est produit par la dissociation d'une liaison azote-carbone après une réaction d'oxydation de la seconde amine aliphatique au-dessus d'une électrode et non par l'intermédiaire d'un cation radical, et
dans lequel le second radical neutre interagit en outre avec la première amine aliphatique pour produire des premiers radicaux neutres supplémentaires.

2. Procédé d'analyse biomoléculaire selon la revendication 1, dans lequel la première amine aliphatique est la tripropylamine.

3. Procédé d'analyse biomoléculaire selon la revendication 1, dans lequel la seconde amine aliphatique est la dipropylisobutylamine.

4. Procédé d'analyse biomoléculaire selon la revendication 1, dans lequel la seconde amine aliphatique est la diisopropyléthylamine.

5. Procédé d'analyse biomoléculaire selon la revendication 1, dans lequel la seconde amine aliphatique est la 1-allylpipéridine.

6. Procédé d'analyse biomoléculaire selon la revendication 1, dans lequel la seconde amine aliphatique est la 1-allylpyrrolidine.

7. Procédé d'analyse biomoléculaire selon la revendication 1, dans lequel un rapport de quantités entre la première amine aliphatique et la seconde amine aliphatique est ajusté en fonction d'un type de l'objet de test.

8. Dispositif d'analyse biomoléculaire, comprenant :
une cellule à circulation (101) ;
une électrode de travail (102), une contre-électrode (103) et une électrode de référence (104) qui sont disposées à l'intérieur de la cellule à circulation (101) ; et
une unité de commande (105) destinée à effectuer un traitement d'analyse consistant à : faire circuler une solution dans la cellule à circulation (101), la solution contenant un objet de test, un support magnétique (106) au-dessus de la surface duquel est formé un complexe comprenant un anticorps qui est marqué par un agent luminescent et qui reconnaît l'objet de test, ainsi qu'une première amine aliphatique qui ne comporte qu'une chaîne alkyle linéaire et une seconde amine aliphatique en tant que premier et second auxiliaires de réaction destinés à favoriser une réaction de l'agent luminescent,
dans lequel la seconde amine aliphatique comporte au moins une chaîne alkyle ayant une structure ramifiée, ou la seconde amine aliphatique comporte une liaison insaturée dans une chaîne alkyle, ou la seconde amine aliphatique comporte un groupe hydroxyle ou un atome d'halogène dans une chaîne alkyle ;
capturer le support magnétique (106) au-dessus de l'électrode de travail (102) au moyen d'un champ magnétique généré par un générateur de force magnétique (107) ; exciter la luminescence de l'agent luminescent en appliquant une tension à l'électrode de travail (102) ; et mesurer une quantité de luminescence de l'agent luminescent au moyen d'un dispositif de mesure,
dans lequel la solution contient un premier radical neutre et un second radical neutre et agit sur la luminescence de l'agent luminescent,
dans lequel le premier radical neutre est produit à partir de la première amine aliphatique, qui est l'auxiliaire de réaction, par l'intermédiaire d'un cation radical et agit sur la luminescence de l'agent luminescent,
dans lequel le second radical neutre est produit par la dissociation d'une liaison azote-carbone après une réaction d'oxydation de la seconde amine aliphatique au-dessus d'une électrode et non par l'intermédiaire d'un cation radical,
dans lequel le second radical neutre interagit en outre avec la première amine aliphatique pour produire des premiers radicaux neutres supplémentaires.
